# EUROPEAN PATENT APPLICATION

(11) **EP 4 011 408 A1**
(43) Date of publication of application: **15.06.2022**
(21) Application number: 21169831.1
(22) Date of filing: 22.04.2021
(51) Int. Cl.: A61L 9/20, A61L 9/22, F21V 33/00

(54) **LAMP HAVING AIR PURIFICATION AND STERILIZATION FUNCTIONS**

(30) Priority: 11.12.2020 CN 202011446948
(71) Applicant: Dozen Energy Technology Co., Ltd., Guangdong (CN)
(72) Inventor: Ma, Aimin, Dongguan (CN)
(74) Representative: Cabinet Chaillot

(57) **Abstract**

The present invention discloses a lamp having air purification and sterilization functions, comprising a cabinet (1), a light source mechanism fixedly mounted on the cabinet (1), a sterilization mechanism for purifying and sterilizing a fluid flow, a fluid inlet (2) and a fluid outlet (3) disposed on the cabinet (1), a circulation mechanism for circulating the fluid flow from the fluid inlet (2) to the fluid outlet (3), and a fluid flowing path extending from the fluid inlet (2), passing through the sterilization mechanism, and reaching the fluid outlet (3). The present invention utilizes the sterilization mechanism to sterilize bacteria and viruses suspending in the air, and ionizes the air to generate plasma according to a point discharge principle; the plasma can effectively inhibit the breeding of pathogenic bacteria in a public place; furthermore, the cabinet can be utilized to mount the lamp at the top of an indoor space, and the light source mechanism under the cabinet illuminates the space, thereby overcoming the defect that an air purifier or an ultraviolet sterilization lamp cannot be mounted due to the limited indoor space, and having the advantage of diversified use functions.

## Description

### TECHNICAL FIELD

The present invention relates to the technical field of illumination lamps, and more particularly to a lamp having air purification and sterilization functions.

### BACKGROUND

At present, two lamps are generally used at the places such as a house, a school, an office, a factory, an emporium, and various entertainment places; one is a ceiling-mounted lamp which, as the name implies, is mounted with a bottom completely attached on a ceiling because the ceiling above the lamp is flat, and the light source includes an ordinary white bulb, a fluorescent lamp, a high strength gas discharge lamp, a halogen tungsten lamp, an LED and the like, wherein the LED ceiling-mounted lamp is the most popular ceiling-mounted lamp on the market at present; the other one is a ceiling lamp which is an illumination lamp embedded in the ceiling and emits light downward. The mounting of the lamp will not destroy the artistic perfect unity of the ceiling; the biggest feature thereof is that the overall unity and perfection of architectural decoration can be maintained; the structure has the following advantages: the light source is hidden in the architectural decoration, and the LED light source is not exposed outside, and will not stimulate eyes and skin.

However, the lamp at present generally only has lighting function. At densely populated public places such as a school, a factory, an emporium, an office and the like, the air quality is comparatively poor, and bacteria and viruses are easy to spread in the air. Due to the limited space, an air purifier or an ultraviolet sterilization lamp cannot be mounted. Especially in the outbreak of Corona Virus Disease 2019 (COVID-19) in early 2020, the problem of sterilization device shortage in public places emerges abruptly. The shortage of sterilization device is easy to cause large scale infection at the densely populated public places such as a school, a factory, an emporium, an office and the like. Therefore, it is necessary to overcome the above problem.

### SUMMARY

The objective of the present invention is to provide a lamp having air purification and sterilization functions which has the advantage of diversified use functions and solves the problem proposed in the background.

To achieve the above objective, the present invention provides the following technical solution: a lamp having air purification and sterilization functions, including a cabinet, a light source mechanism fixedly mounted on the cabinet, a sterilization mechanism for purifying and sterilizing a fluid flow, a fluid inlet and a fluid outlet disposed on the cabinet, a circulation mechanism for circulating the fluid flow from the fluid inlet to the fluid outlet, and a fluid flowing path extending from the fluid inlet, passing through the sterilization mechanism, and reaching the fluid outlet.

Preferably, the sterilization mechanism includes a plasma generator and an ionization apparatus; the ionization apparatus is fixedly mounted in the cabinet; a plurality of ionization portions are integrally disposed on the ionization apparatus; the ionization portions have a wedge shape; and the plasma generator is electrically connected to the ionization apparatus.

Preferably, the sterilization mechanism includes a negative ion generator and an ionization apparatus; the ionization apparatus is fixedly mounted in the cabinet; a plurality of ionization portions are integrally disposed on the ionization apparatus; the ionization portions have a wedge shape; and the negative ion generator is electrically connected to the ionization apparatus.

Preferably, the sterilization mechanism includes a UV lamp tube; the UV lamp tube is fixedly mounted in the cabinet on one side adjacent to the fluid inlet; and the UV lamp tube is a shortwave ultraviolet lamp tube with a wavelength of 200nm-280nm.

Preferably, the circulation mechanism includes a fan and a circulation air duct; the fan is fixedly mounted in the cabinet; and the circulation air duct is respectively in communication with the fluid outlet and the fan.

Preferably, the light source mechanism includes a light source plate; and the light source plate is fixedly mounted on a lower end surface of the cabinet.

Compared with the prior art, the present invention has the following beneficial effects:
The present invention is provided with a sterilization mechanism which is configured to purify and sterilize a fluid flow; the sterilization mechanism includes a plasma generator, a negative ion generator, an ionization apparatus, and an UV lamp tube; the ionization apparatus is fixedly mounted in the cabinet; a plurality of ionization portions are integrally disposed on the ionization apparatus; the ionization portions have a wedge shape; the plasma generator or the negative ion generator is respectively electrically connected to a circuit mechanism and the ionization apparatus; the UV lamp tube is electrically connected to the circuit mechanism; the UV lamp tube is fixedly mounted in the cabinet; the UV lamp tube is a shortwave ultraviolet lamp tube with a wavelength of 200nm-280nm; the present invention utilizes the plasma generator, the negative ion generator, and the UV lamp tube to sterilize bacteria and viruses suspending in the air, and ionizes the air to generate plasma according to a point discharge principle; the plasma can effectively inhibit the breeding of pathogenic bacteria in a public place; furthermore, the cabinet can be utilized to mount the lamp at the top of an indoor space, and the light source mechanism under the cabinet illuminates the space, thereby overcoming the defect that an air purifier or an ultraviolet sterilization lamp cannot be mounted due to the limited indoor space, and achieving the effect of diversified use functions.

### BRIEF DESCRIPTION OF THE DRAWINGS

Fig. 1 is a structural schematic view of the present invention;
Fig. 2 is an internal structural schematic view of a first embodiment of the present invention;
Fig. 3 is an exploded structural schematic view of the first embodiment of the present invention;
Fig. 4 is a structural schematic view of the ionization apparatus according to the present invention;
Fig. 5 is an internal structural schematic view of a second embodiment of the present invention; and
Fig. 6 is an exploded structural schematic view of the second embodiment of the present invention.

Reference signs and names in the figures are as follows:
1, cabinet; 2, fluid inlet; 3, fluid outlet; 4, plasma generator; 5, ionization apparatus; 6, ionization portion; 7, negative ion generator; 8, UV lamp tube; 9, fan; 10, circulation air duct; and 11, light source plate.

### DETAILED DESCRIPTION OF THE EMBODIMENTS

The technical solutions in the embodiments of the present invention will be clearly and completely described hereafter in combination with the drawings in the embodiments of the present invention. It is apparent that the described embodiments are only a part of the embodiments of the present invention, but not the whole. On the basis of the embodiments in the present invention, all the other embodiments obtained by a person skilled in the art without involving an inventive effort are all concluded in the protection scope of the present invention.

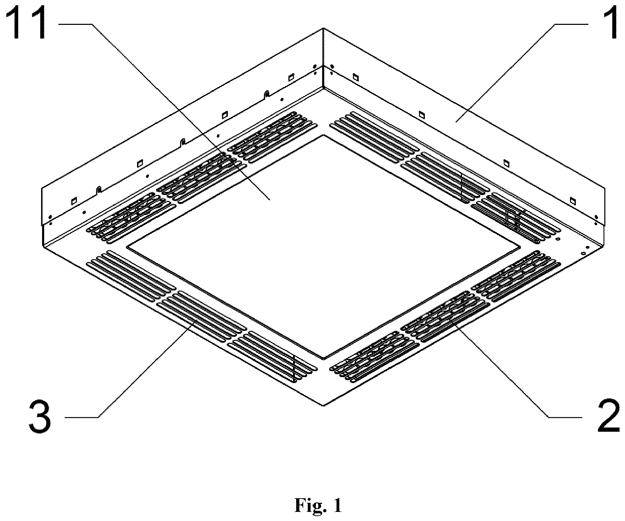

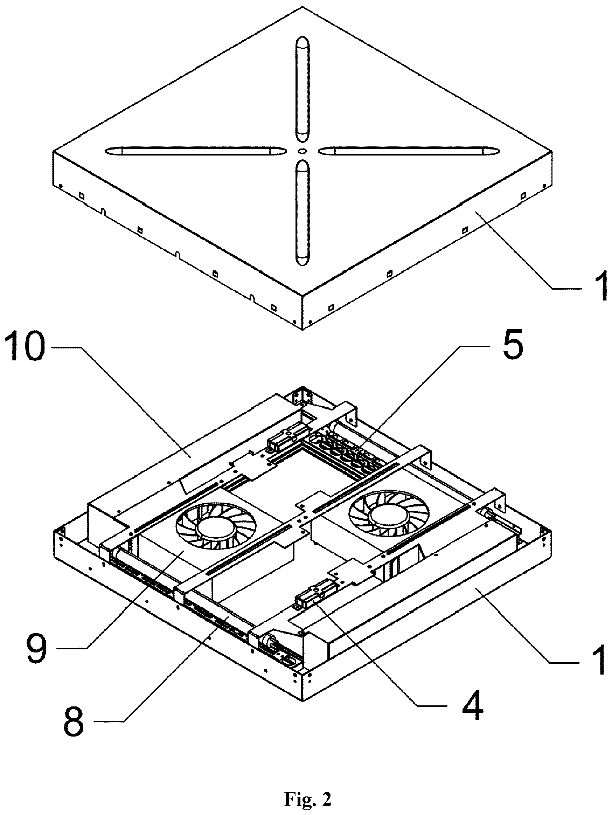

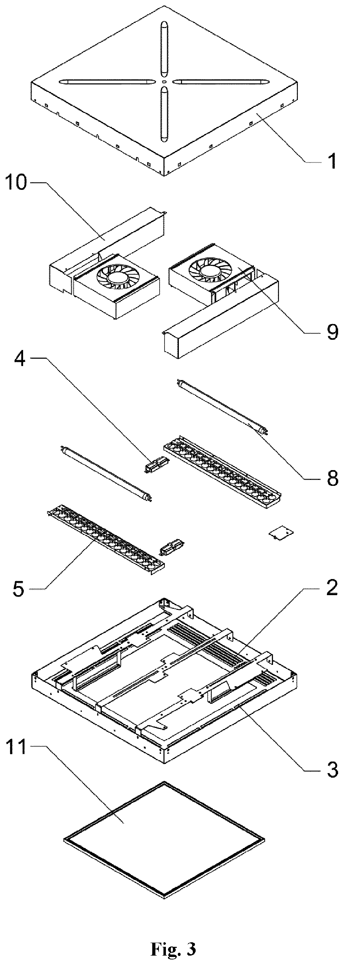

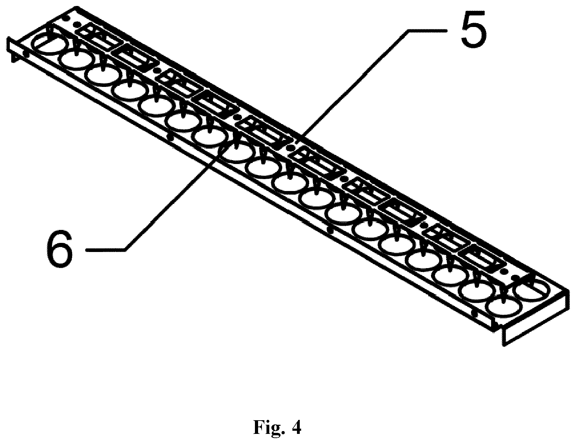

With reference to figures 1-4, the present invention provides a first embodiment: a lamp having air purification and sterilization functions, including a cabinet 1, a light source mechanism fixedly mounted on the cabinet 1, a sterilization mechanism for purifying and sterilizing a fluid flow, a fluid inlet 2 and a fluid outlet 3 disposed on the cabinet 1, a circulation mechanism for circulating the fluid flow from the fluid inlet 2 to the fluid outlet 3, and a fluid flowing path extending from the fluid inlet 2, passing through the sterilization mechanism, and reaching the fluid outlet 3.

More specifically, the sterilization mechanism includes a plasma generator 4 and an ionization apparatus 5; the ionization apparatus 5 is fixedly mounted in the cabinet 1; a plurality of ionization portions 6 are integrally disposed on the ionization apparatus 5; the ionization portions 6 have a wedge shape; and the plasma generator 4 is electrically connected to the ionization apparatus 5; the plasma generator 4 is utilized to enable the ionization apparatus 5 to generate plasma; active particles such as high energy electrons, free radicals and the like are utilized to react with a pollutant in the air, such that pollutant molecules are decomposed in a very short time, thus achieving the objective of air purification by means of subsequent various reactions.

More specifically, the sterilization mechanism includes a UV lamp tube 8; the UV lamp tube 8 is fixedly mounted in the cabinet 1; and the UV lamp tube 8 is a shortwave ultraviolet lamp tube with a wavelength of 200nm-280nm; ultraviolet radiation is utilized to photolyse and denature bacteria protein, and destroy the amino acid, nucleic acid and enzyme to sterilize the bacteria; furthermore, when the ultraviolet passes through the air, the oxygen in the air is ionized to generate ozone, thereby enhancing the sterilization effect.

More specifically, the circulation mechanism includes a fan 9 and a circulation air duct 10; the fan 9 is fixedly mounted in the cabinet 1; and the circulation air duct 10 is respectively in communication with the fluid outlet 3 and the fan 9; a fluid flow enters the fluid inlet 2 under the drive of the fan 9, is then purified and sterilized by the sterilization mechanism, next enters the circulation air duct 10, and finally circulates from the fluid outlet 3 to an indoor space.

More specifically, the light source mechanism includes a light source plate 11; and the light source plate 11 is fixedly mounted on a lower end surface of the cabinet 1.

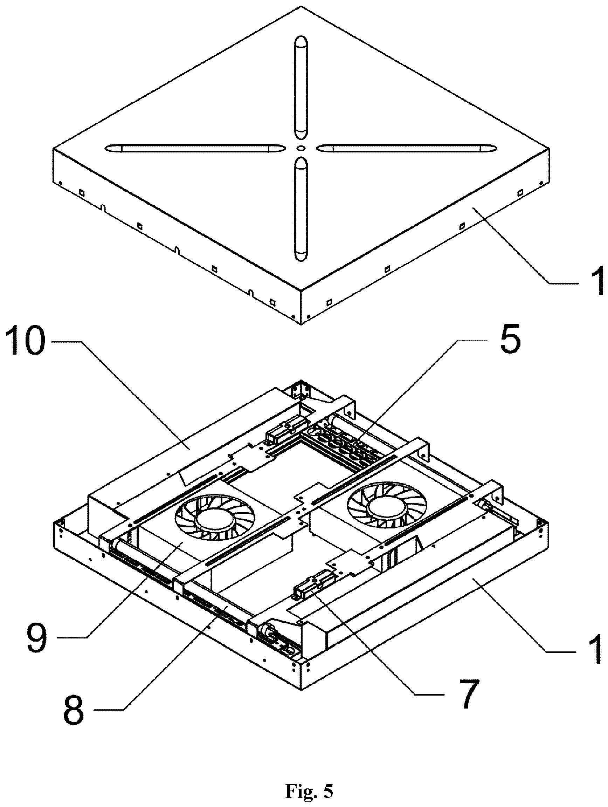

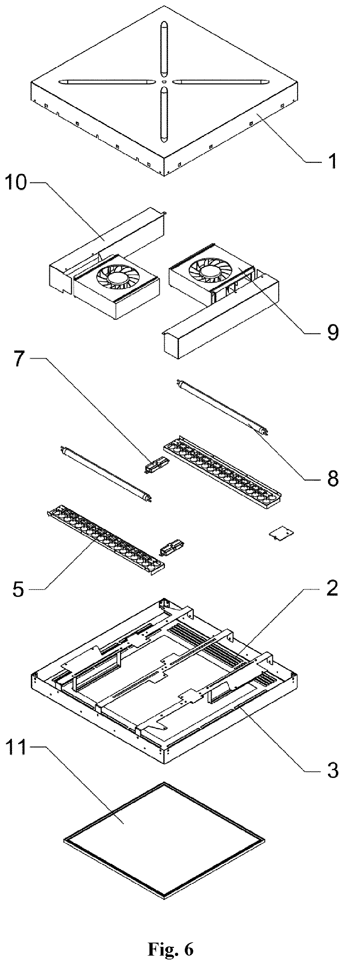

With reference to figures 5-6, the present invention provides a second embodiment; the second embodiment is basically the same as the first embodiment; the differences are: the sterilization mechanism includes a negative ion generator 7 and an ionization apparatus 5; the ionization apparatus 5 is fixedly mounted in the cabinet 1; a plurality of ionization portions 6 are integrally disposed on the ionization apparatus 5; the ionization portions 6 have a wedge shape; the negative ion generator 7 is electrically connected to the ionization apparatus 5; the negative ion generator 7 is utilized to generate negative air ions; the negative air ion is formed by adding a pair of free electrons to an oxygen molecule, and carries negative charges, thereby having an extraordinary binding ability; therefore, dusts, vapor, bacteria, and viruses which float in a house and generally carry positive charges are gathered together, lose the ability of freely floating in the air, and quickly fall down, so as to purify the air and the environment.

### Operating principle:

Plasma sterilization refers to: utilizing the plasma generator 4 to enable the ionization apparatus 5 to generate plasma, utilizing the active particles such as high energy electrons, free radicals and the like to react with a pollutant in the air, such that pollutant molecules are decomposed in a very short time, thus achieving the objective of air purification by means of subsequent various reactions.

Negative ion sterilization refers to: utilizing the negative ion generator 7 to generate negative air ions, wherein the negative air ion is formed by adding a pair of free electrons to an oxygen molecule, and carries negative charges, thereby having an extraordinary binding ability; gathering dusts, vapor, bacteria, and viruses which float in a house and generally carry positive charges together, such that the dusts, vapor, bacteria, and viruses lose the ability of freely floating in the air and quickly fall down, so as to purify the air and the environment.

Ultraviolet sterilization refers to: utilizing ultraviolet radiation to photolyse and denature bacteria protein, and destroying the amino acid, nucleic acid and enzyme to sterilize the bacteria. Furthermore, when the ultraviolet passes through the air, the oxygen in the air is ionized to generate ozone, thereby enhancing the sterilization effect.

The present invention uses the above three methods to sterilize bacteria and viruses suspending in the air, generates plasma according to a point discharge principle so as to purify the dusts and pathogenic bacteria suspending in the air, and can effectively inhibit the breeding of pathogenic bacteria in a public place; the fan 9 and the circulation air duct 10 enable a great deal of treated clean air to quickly circulate and flow in a house; furthermore, the cabinet 1 can be utilized to mount the lamp at the top of an indoor space, and the light source mechanism under the cabinet 1 illuminates the space, thereby overcoming the defect that an air purifier or an ultraviolet sterilization lamp cannot be mounted due to the limited indoor space, and achieving the effect of diversified use functions.

The fluid flowing through the sterilization mechanism is generally the air, and can also be a gas or different combinations of multiple gases. The fluid may include an additive configured to improve the performance of the sterilization mechanism or reduce the affect of the output fluid on an object in alignment with the fluid outlet.

For a person skilled in the art, it is apparent that the present invention is not limited to the details of the above exemplary embodiments, and the present invention can be implemented in other specific forms without departing from the spirit or basic features of the present invention. Therefore, no matter from which point of view, the embodiments should all be regarded as exemplary and non-limiting. The scope of the present invention is defined by the appended claims rather than the above description, and therefore it is intended that all the changes which fall within the meaning and scope of equivalents of the claims are embraced in the present invention. Any reference sign in the claims should not be construed as limiting the related claims.

## Claims

1. A lamp having air purification and sterilization functions, **characterized in that** the lamp comprises:
a cabinet (1);
a light source mechanism fixedly mounted on the cabinet (1);
a sterilization mechanism for purifying and sterilizing a fluid flow;
a fluid inlet (2) and a fluid outlet (3) disposed on the cabinet (1);
a circulation mechanism for circulating the fluid flow from the fluid inlet (2) to the fluid outlet (3); and
a fluid flowing path extending from the fluid inlet (2), passing through the sterilization mechanism, and reaching the fluid outlet (3).

2. A lamp having air purification and sterilization functions according to claim 1, **characterized in that** the sterilization mechanism comprises a plasma generator (4) and an ionization apparatus (5); the ionization apparatus (5) is fixedly mounted in the cabinet (1); a plurality of ionization portions (6) are integrally disposed on the ionization apparatus (5); the ionization portions (6) have a wedge shape; and the plasma generator (4) is electrically connected to the ionization apparatus (5).

3. A lamp having air purification and sterilization functions according to claim 1, **characterized in that** the sterilization mechanism comprises a negative ion generator (7) and an ionization apparatus (5); the ionization apparatus (5) is fixedly mounted in the cabinet (1); a plurality of ionization portions (6) are integrally disposed on the ionization apparatus (5); the ionization portions (6) have a wedge shape; and the negative ion generator (7) is electrically connected to the ionization apparatus (5).

4. A lamp having air purification and sterilization functions according to claim 1, **characterized in that** the sterilization mechanism comprises a UV lamp tube (8); the UV lamp tube (8) is fixedly mounted in the cabinet (1); and the UV lamp tube (8) is a shortwave ultraviolet lamp tube with a wavelength of 200nm-280nm.

5. A lamp having air purification and sterilization functions according to claim 2, **characterized in that** the sterilization mechanism comprises a UV lamp tube (8); the UV lamp tube (8) is fixedly mounted in the cabinet (1); and the UV lamp tube (8) is a shortwave ultraviolet lamp tube with a wavelength of 200nm-280nm.

6. A lamp having air purification and sterilization functions according to claim 3, **characterized in that** the sterilization mechanism comprises a UV lamp tube (8); the UV lamp tube (8) is fixedly mounted in the cabinet (1); and the UV lamp tube (8) is a shortwave ultraviolet lamp tube with a wavelength of 200nm-280nm.

7. A lamp having air purification and sterilization functions according to claim 1, **characterized in that** the circulation mechanism comprises a fan (9) and a circulation air duct (10); the fan (9) is fixedly mounted in the cabinet (1); and the circulation air duct (10) is respectively in communication with the fluid outlet (3) and the fan (9).

8. A lamp having air purification and sterilization functions according to claim 1, **characterized in that** the light source mechanism comprises a light source plate (11); and the light source plate (11) is fixedly mounted on a lower end surface of the cabinet (1).
